# EUROPEAN PATENT APPLICATION

(11) **EP 1 491 167 A1**
(43) Date of publication of application: **29.12.2004**
(21) Application number: 04253607.8
(22) Date of filing: 16.06.2004
(51) Int. Cl.: A61F 5/00, A61F 2/00, A61B 17/12

(54) **Implantable band with non-mechanical attachment mechanism**

(30) Priority: 27.06.2003 US 483353 P; 30.09.2003 US 676368
(71) Applicant: ETHICON ENDO-SURGERY, INC., Cincinnati, Ohio 45242 (US)
(72) Inventor: Byrum, Randal T., Milford, Ohio 45150 (US); Nuchols, Richard P., Loveland Ohio 45140 (US)
(74) Representative: Tunstall, Christopher Stephen

(57) **Abstract**

An implantable band for placement around an anatomical passageway, such as the stomach or other lumen, has a non-mechanical attachment mechanism, particularly an adjustable gastric band, includes a hook and loop fastener, magnetic material, adhesive and energy bonded material.

## Description

### Related Applications

This application claims the priority of provisional patent application serial number 60/483,353 filed on June 27, 2003, the disclosure of which is incorporated herein by reference. The application also incorporates by reference the following co-pending United States Patent Applications filed of even date herewith: Provisional Application Serial No. 60/483,353 (*Implantable Band Having Improved Attachment Mechanism,* inventors: Byrum, Jambor, Albrecht); Application Serial No. 10/677,088 (*Implantable Band with Transverse Attachment Mechanism,* inventors: Byrum, Jambor, Crawford); Provisional Application Serial No. 60/483,353 (*Implantable Band with Attachment Mechanism,* inventors: Byrum, Jambor, Conlon, Crawford, Harper, Spreckelmeier); and Provisional Application Serial No. 60/507,612 (*Implantable Band with Deformable Attachment Mechanism,* inventors: Byrum, Wiley, Conlon, Fender).

### Technical Field

This present invention relates generally to a surgically implantable band for encircling a anatomical passageway, and is particularly directed to an adjustable gastric band for encircling the stomach for the control of obesity. The invention will be specifically disclosed in connection with an improved attachment mechanism for an adjustable gastric band.

### Background Of The Invention

Since the early 1980s, adjustable gastric bands have provided an effective alternative to gastric bypass and other irreversible surgical weight loss treatments for the morbidly obese. The gastric band is wrapped around an upper portion of the patient's stomach, forming a stoma that is less than the normal interior diameter of the stomach that restricts food passing from an upper portion to a lower digestive portion of the stomach. When the stoma is of the appropriate size, food held in the upper portion of the stomach provides a feeling of fullness that discourages overeating.

In addition to a latched position to set the diameter of the gastric band, adjustability of gastric bands is generally achieved with an inwardly directed inflatable balloon, similar to a blood pressure cuff, into which fluid, such as saline, is injected through a fluid injection port to achieve a desired diameter. The balloon is typically deflated or only partially inflated when first placed in the body to allow for body adjustments and healing around the new band site. Since adjustable gastric bands may remain in the patient for long periods of time, the fluid injection port is typically installed subcutaneously to avoid infection, for instance in front of the sternum. Following the initial implantation, the surgeon may adjust the band by loosing or tightening depending on the patients' needs. Adjusting the amount of fluid in the adjustable gastric band is achieved by inserting a Huber tip needle through the skin into a silicone septum of the injection port. Once the needle is removed, the septum seals against the hole by virtue of compressive load generated by the septum. A flexible conduit communicates between the injection port and the adjustable gastric band.

An attachment mechanism for the adjustable gastric band has to provide an initial sizing of the stoma of the stomach. One generally known attachment is to suture ends of the adjustable gastric band. Another generally known attachment includes one end of the gastric band terminating in a flexible conduit that has a flared portion that is drawn through an opening in a second end of the gastric band and then sutured to the encircling band portion - securing the band to the stomach. After the sutures are in place, the injection port is anchored at a convenient location.

While these known approaches are effective in securing the gastric band, further improvements are desired that simplify the clinical implantation procedure, that provide long-term reliability, and that facilitate readjustment or removal.

While sutures have been relied on as the most positive connection in the past, it is desirable to have a secure attachment that does not require sutures, yet does not require a large force to create the secure attachment. Otherwise, it may be difficult to adequately grip and perform the attachment with laparoscopic instruments. Consequently, a significant need exists for an adjustable gastric band having an improvement attachment mechanism.

### Summary of The Invention

The present invention addresses these and other problems in the prior art, by providing an adjustable gastric band device that is engaged with less force, thereby facilitating implementation with laparoscopic instruments, yet the attachment remains secure over long term use.

A general object of this invention is to provide an adjustable gastric band having a non-mechanical attachment mechanism.

Another object of this invention is to provide a readily reversible adjustable gastric band which can be fastened and unfastened without reducing the holding strength of the attachment mechanism.

Another object of this invention is to provide an adjustable gastric band having a secure fastening mechanism which can be fastened and unfastened without minimizing the strength of the attachment mechanism.

To achieve the foregoing and other objects, and in accordance with the purposes of the present invention as described herein, there are described adjustable gastric bands with non-mechanical attachment mechanisms connecting the two ends together. The non-mechanical attachment mechanisms include a hook and loop attachment mechanism, a magnetic attachment mechanism, an adhesive attachment mechanism and the use of energy bonding.

Further novel features and other objects of the present invention will become apparent from the following detailed description, discussion and the appended claims, taken in conjunction with the drawings.

### Brief Description Of The Figures

The accompanying drawings, which are incorporated in and constitute a part of this specification, illustrate embodiments of the invention, and, together with the general description of the invention given above, and the detailed description of the embodiments given below, serve to explain the principles of the present invention.

FIG. 1 is a diagrammatic drawing showing an adjustable gastric band wrapped around an upper part of a stomach.

FIG. 2 is a cross sectional view of the adjustable gastric band of FIG 1 taken along line 2-2.

FIG. 3 is a top, plan view of an adjustable gastric band constructed in accordance with the present invention having a hook and loop attachment mechanism.

FIG. 4 is a top, plan view of an alternate embodiment of the adjustable gastric band shown in Fig. 3.

FIG. 5 is a top, plan view of another embodiment of an adjustable gastric band constructed in accordance with the present invention having a magnetic attachment mechanism.

FIG. 6 is a fragmentary, enlarged, perspective view of the two end portions of an adjustable gastric band constructed in accordance with the present invention having an adhesive attachment mechanism.

FIG. 7 is a fragmentary, enlarged, perspective view of the two end portions of an adjustable gastric band constructed in accordance with the present invention in which the two ends have been energy bonded together.

Reference will now be made in detail to the present preferred embodiment of the invention, an example of which is illustrated in the accompanying drawings.

### Detailed Description of Embodiments of the Invention

In the following description, like reference characters designate like or corresponding parts throughout the several views. Also, in the following description, it is to be understood that terms such as front, back, inside, outside, and the like are words of convenience and are not to be construed as limiting terms. Terminology used in this patent is not meant to be limiting insofar as devices described herein, or portions thereof, may be attached or utilized in other orientations. Referring in more detail to the drawings, the invention will now be described.

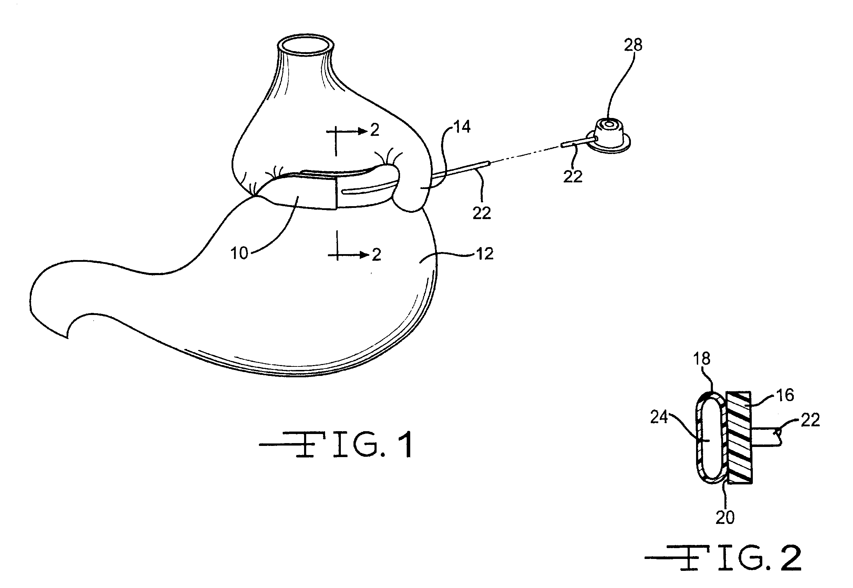

Referring to Fig. 1, an adjustable gastric band 10 is shown wrapped around an upper portion of a stomach 12, kept in place by attaching the two ends together and extending a portion 14 of the stomach 12 over the adjustable gastric band 10 by suturing portion 14 to the stomach. Referring also to Fig. 2, the adjustable gastric band 10 includes a non-extensible strap 16 and an inflatable balloon 18, made of a medical grade silicone polymer or any other suitable material, is carried by the inner surface 20 of the strap 16. The balloon 18 may be secured to the inner surface 20 in any well known manner, or even made of unitary construction with the strap 16, although the strap 16 may typically be formed of a different material.

One end of a flexible conduit 22 is in fluid communication with the internal cavity 24 of the balloon 18, with the other end being in fluid communication with an internal cavity (not shown) of a remote injection port 26. The remote injection port 26 includes a silicone septum 28. At the time the adjustable gastric band 10 is implanted around a portion of the stomach, the remote injection port 26 is also implanted at a suitable location, usually within the rectus sheaths, for transcutaneous access via a Huber needle.

As is well known, the internal cavity 24, the flexible conduit 22 and the internal cavity of the remote injection port 26 are preferably at least partially filled with a physiologically compatible fluid, such as a saline solution. Postoperative adjustment of the perimeter enclosed by the balloon 18, and therefore the size of the stoma, is accomplished by addition or removal of fluid from the interior cavity 24 of the balloon 18 by inserting a Huber needle percutaneously into the silicone septum 28 of the injection port 18.

As is well known in the field the adjustable gastric band 10 may be made from any suitable medically compatible material having sufficient strength necessary for a particular laparoscopic surgery or particular patient.

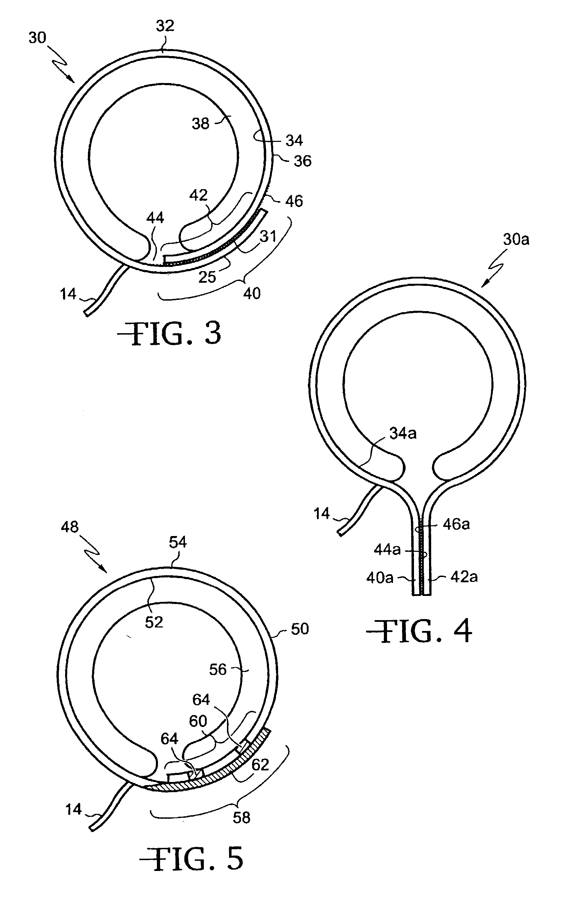

As mentioned above, the two ends of the adjustable gastric band 10 are attached together (the specific attachment mechanism structure is not illustrated in FIG. 1). The present invention is directed to various embodiments of non-mechanical attachment mechanisms for connecting the two ends together. The general construction of adjustable gastric band 10 shown in FIGS. 1 and 2 and described above is common to the embodiments illustrated in FIGS. 3-7, with the embodiments differing by the specific attachment mechanisms. It is noted that the practice of the present invention may be used with any gastric band, and is not limited to use with an adjustable gastric band having the exact features described above or below.

Turning now to FIG. 3, the adjustable gastric band 30 includes an elongated strap 32 extending in what is referred to herein as the longitudinal direction, even though when implanted the adjustable gastric band 30 has an arcuate configuration. The strap 32 includes an inner surface 34 and an outer surface 36, with the balloon 38 extending inwardly from adjacent the inner surface 34. The adjustable gastric band 30 includes a first end portion 40 which overlaps a second end portion 42, with the inner surface 34 of the adjustable gastric band 30 at the first end portion 40 being disposed adjacent the outer surface 36 of the adjustable gastric band 30 at the second end 42 portion.

The first and second end portions 40, 42 are secured together by a non-mechanical attachment mechanism. For the purposes of this description, non-mechanical attachment mechanism refers to a mechanism for latching which is not readily visible with the naked eye. These include such elements as magnets, adhesives, material welding, hook-and-loop, and the like. Prior art bands utilize geometry-based features which create interferences and connections. In the embodiment depicted in Fig. 3, the non-mechanical attachment mechanism is a hook and loop fastener carried by the adjacent surfaces of the first and second end portions 40, 42. The inner surface 34 of the end portion 40 carries either the hook material or loop material, indicated at 44, with the outer surface 36 of the second end portion 42 carrying the corresponding mating material, either the loop or hook material, indicated at 46. The fastening characteristics of hook and loop material is well known, with the hook material engaging the loop material at a very small, micro level, having no specific alignment between individual hooks and individual loops, with numerous hooks and loops per square inch of material producing substantial load sharing with no single hook and loop carrying a substantial portion of the total load. Even though the operation of hook and loop material involves hooks partially extending around loops, this attachment mechanism is considered herein as being non-mechanical.

The hook and loop material fasteners 44, 46, are attached to the respective surfaces of the first and second end portions 34, 36 by any suitable method, such as for example by a physiologically compatible adhesive. The hook and loop material 44, 46, may be made of any medically compatible material, including for example stainless steel. The first and second end portions 40a, 42a overlap a length which is at least sufficient for the two end portions to be held together for the particular non-mechanical attachment mechanism used. By way of example, the overlap length for the hook and loop non-mechanical attachment mechanism illustrated in FIG. 3 may be approximately 2.5 cm.

Hook and loop fastener 44, 46 allow easy and quick fastening of the ends of adjustable gastric band with sufficient holding strength to remain in place during post operative adjustments to balloon 18, but which can be removed or adjusted by a surgeon when desired.

FIG. 4 illustrates an alternate embodiment of the adjustable gastric band embodiment of FIG. 3. The adjustable gastric band 30a includes a hook and loop non-mechanical mechanism carried by the adjacent surfaces of the first and second end portions 40a and 42a. In this embodiment the inner surface 34a of the adjustable gastric band 30a at the second end portion 42a carries the corresponding mating material, either the loop or hook material 46a to engage the hook or loop material 44a carried by the inner surface 34a of the adjustable gastric band 30a at first end portion 40a. In this embodiment, when the first and second end portions 40a, 42a are secured together, the inner surface 34a of each are opposed to each other. It is noted that the connected first and second end portions 40a, 42a, may extend outwardly at any angle, such as in the general radial direction depicted in FIG. 4, generally aligned with the center of the area enclosed by the adjustable gastric band 30a. As alternate to this alternate embodiment, either the first or second end portion could extend through an opening formed in the other end portion, or around an edge of the other end portion, such that the outer surface of the end portions oppose each other, with hook and loop material being respectively carried by the opposing surfaces.

The embodiment shown in FIG. 5 includes a magnetic non-mechanical attachment mechanism. The adjustable gastric band 48 includes an elongated strap 50 which has an inner surface 52 and an outer surface 54, with the balloon 56 extending inwardly from adjacent the inner surface 52. The adjustable gastric band 48 includes a first end portion 58 which overlaps a second end portion 60, with the inner surface 52 of the first end portion 58 being disposed adjacent the outer surface 54 of the second end portion 60.

Each of the first and second end portions 58, 60 carry a magnetic material 62, 64, at least one of which is a magnet. As used herein, magnetic material includes a magnet and any material which is attracted to a magnet. The magnetic material 62, 64 may be any suitable medically compatible material. When implanted, the first and second end portions 58, 60, are held together by the magnetic force between the magnetic material, with the magnetic material being selected, sized and located so as to produced sufficient magnetic force therebetween to hold the two end portions 58, 60 together.

As seen in FIG. 5, the entire first end portion 58 is magnetic material 62, secured to the strap 50 in any suitable manner, such as by heat or pressure bonding, adhesive, or mechanical attachment such as sewing or stapling. Alternatively, magnetic material 62 may comprise a shell disposed around the strap 50.

The second end portion 60 is illustrated as carrying spaced apart, discrete members of magnetic material 64, such as strips of magnetic material extending in a transverse direction across the width of the second end portion 60 of the strap 50. The members of magnetic material 64 may extend across the width of the strap 50. The members of magnetic material 64 may be attached to end portion 60 in any suitable manner. The members of magnetic material 64 could be embedded, being enclosed completely by the second end portion 60, such as being disposed in a cavity formed therein or being molded in place.

Any configuration of magnetic material carried by the first and second end portions 58, 60 may be used. For example, both end portions 58, 60 may be strips of magnetic material. Both may carry one or more individual magnetic material inserts. The magnetic material on both end portions 58, 60 may each be one or more magnets (but at least one of which of any mating pair or portion of magnetic material must be a magnet), with the appropriate orientation and alignment of the magnetic poles. The magnetic material may be secured to or in the end portions 58, 60 in any suitable manner. The magnetic material may be flexible or rigid.

Additionally, the end portions 58 and 60, and magnetic material 62, 64 may be arranged in alternate configurations as discussed above with respect to the hook and loop attachment mechanism. For example, the magnetic material on each end portion may be disposed such that the inner surfaces of the end portions are disposed adjacent each other, or such that outer surfaces of the end portions are disposed adjacent each other.

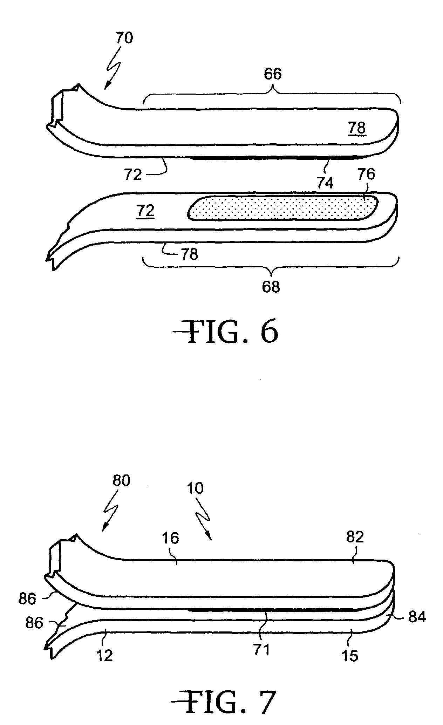

The embodiment shown in FIG. 6 includes an adhesive non-mechanical attachment mechanism. FIG. 6 is an enlarged, fragmentary, perspective view of the first end portion 66 and the second end portion 68 of the adjustable gastric band 70. At least one of the inner surface 72 of the adjustable gastric band 70 at the first end portion 66 and the inner surface 72 of the adjustable gastric band 70 at second end portion 66 carries an adhesive which is suitable to secure the two end portions 66, 68 together. As seen in FIG. 6, the inner surface 72 at first end portion 66 and the inner surface 72 at second end portion each carry an adhesive 74, 76. The adhesive 74, 76 may be any adhesive which cures quickly on contact with each, is effective in bodily fluids, and exhibits sufficient strength, including such as bonding, impact and tensile strength, and sufficient elongation characteristics, to secure the two end portions 66, 68 together, yet allow a surgeon to separate them when desired. Such adhesives include for example silicone based adhesives. Adhesive 74 may be different than adhesive 76, but must function together to cure upon mutual contact. If an adhesive is carried by only one end portion, the adhesive must cure and bond upon contact with the other end portion.

Prior to implantation the adhesive may need to be activated. This may be done chemically, or may be done by removing a protective material which covers the adhesive 74, 76 to keep foreign material out and to prevent accidental contact therebetween.

The adjustable gastric band 70 is not limited to the configuration illustrated in FIG. 6. As with the previously described embodiments, the adjustable gastric band 70 may be configured alternatively be configured such that the end portions 66, 68 overlap longitudinally, or such that the outer surfaces 78 of the end portions 66, 68 are disposed opposing, adjacent each other.

The embodiment shown in FIG. 7 illustrates an adjustable gastric band 80 having a non-mechanical attachment mechanism achieved by energy bonding of the first end portion 82 to the second end portion 84. The inner surface 86 of the adjustable gastric band 80 at first end portion 82 is securely attached to the inner surface 86 of the adjustable gastric band 80 at the second end portion 84 using a variety of known energy bonding techniques, including for example silicone energy bonding. A surgical tool specially designed for energy welding, such as a grasper (not shown), is used by the surgeon to clamp the two end portions 82, 84 together, and apply the vibratory bonding energy required, as known in the art. The present invention contemplates, but is not limited to any generally known energy bonding like RF welding, ultrasonic welding, harmonic welding and the like.

Using the disclosure of the present invention, those skilled in the art will recognize that other appropriate tools or other bonding processes may be used in practicing the embodiments disclosed in FIGS. 6 and 7, such as solvent or heat bonding.

It will become readily apparent to those skilled in the art that the above invention has equal applicability to other types of implantable bands. For example, bands are used for the treatment of fecal incontinence. One such band is described in U.S. Patent 6,461,292 which is hereby incorporated herein by reference. Bands can also be used to treat urinary incontinence. One such band is described in U.S. Patent Application 2003/0105385 which is hereby incorporated herein by reference. Bands can also be used to treat heartburn and/or acid reflux. One such band is described in U.S. Patent 6,470,892 which is hereby incorporated herein by reference. Bands can also be used to treat impotence. One such band is described in U.S. Patent Application 2003/0114729 which is hereby incorporated herein by reference.

Thus, as used herein and in the claims, an implantable band is a band which may be implanted in a position to occlude flow, such as food or body fluids, through an anatomical passageway, such as a stomach or lumen.

In summary, numerous benefits have been described which result from employing the concepts of the invention. The foregoing description of one or more embodiments of the invention has been presented for purposes of illustration and description. It is not intended to be exhaustive or to limit the invention to the precise form disclosed. Obvious modifications or variations are possible in light of the above teachings. The one or more embodiments were chosen and described in order to best illustrate the principles of the invention and its practical application to thereby enable one of ordinary skill in the art to best utilize the invention in various embodiments and with various modifications as are suited to the particular use contemplated. It is intended that the scope of the invention be defined by the claims appended hereto.

## Claims

1. An implantable band for treatment of a medical condition, the band, comprising
(a) a strap configured to encircle an anatomical passageway, said strap having an inner and outer surface;
(b) first and second end portions disposed at either end of said strap, said first and second end portions configured to be attached to each other so as to secure said strap encircling the anatomical passageway, said first and second end portions including respective inner and outer surfaces which correspond to said inner and outer surfaces of said strap;
(c) at least one of said first and second end portions comprising a non-mechanical attachment mechanism configured to attach said first end portion to said second end portion so as to secure said strap adjacent the anatomical passageway.

2. The band of claim 1, wherein said first end portion carries either hook or loop material and said second end portion carries the other of hook or loop material.

3. The band of claim 1, wherein said inner surface of said first end portion carries either hook or loop material and said outer surface of said second end portion carries the other of hook or loop material.

4. The band of claim 1, wherein said inner surface of said first end portion carries either hook or loop material and said inner surface of said second end portion carries the other of hook or loop material.

5. The band of claim 1, wherein said non-mechanical attachment mechanism comprises magnetic material.

6. The band of claim 5, wherein said first and second end portions comprise said magnetic material.

7. The band of claim 6, wherein at least one of said first and second end portions comprise a magnet.

8. The band of claim 5, wherein at least one of said first and second end portions comprise magnetic material embedded therein.

9. The band of claim 1, wherein said non-mechanical attachment mechanism comprises adhesive.

10. The band of claim 1, wherein one of said inner and outer surfaces of said first end portion carries a first adhesive and one of said inner and outer surfaces of said second end portion carries a second adhesive, whereby said first and second adhesives adhere to each other upon mutual contact.
